# EUROPEAN PATENT APPLICATION

(11) **EP 1 810 671 A1**
(43) Date of publication of application: **25.07.2007**
(21) Application number: 05806145.8
(22) Date of filing: 08.11.2005
(51) Int. Cl.: A61K 31/192, A61K 31/216, A61P 25/28, A61P 43/00, A23L 1/30

(54) **AGENT FOR RECOVERY FROM CEREBRAL FATIGUE**

(30) Priority: 09.11.2004 JP 2004324516; 24.02.2005 JP 2005048198
(71) Applicant: Kao Corporation, Chuo-ku Tokyo 103-8210 (JP)
(72) Inventor: SADACHI, Hidetoshi, Kao Corporation Research Labs, Tokyo 1318501 (JP); NAGASHIMA, Yoshinao, Kao Corporation Research Labs, Tokyo 1318501 (JP); WATANABE, Takuya, Kao Corporation Research Labs, Tokyo 1318501 (JP)
(74) Representative: HOFFMANN EITLE
(86) International application number: PCT/JP2005/020432
(87) International publication number: WO 2006/051773

(57) **Abstract**

The present invention relates to a recuperative agent for cerebral fatigue and a recuperative food for cerebral fatigue, each including as active ingredients one or more members selected from the group consisting of chlorogenic acids, caffeic acid, ferulic acid, and pharmaceutically acceptable salts thereof.

According to the present invention, recuperation from the cerebral fatigue such as decreased calculating ability based on cerebral fatigue can be achieved.

## Description

### Technical Field of the Invention

The present invention relates to a recuperative agent or a recuperative food for the fatigue caused by mental activities intrinsic to humans (e.g., calculating ability), specifically for cerebral fatigue.

### Background of the Invention

Fatigue is usually classified roughly into mental fatigue and physical fatigue. Inmodern society, the influence of mental fatigue is growing more serious than that of physical fatigue. A variety of nutritional supplements, such as vitamins and minerals, may indeed be used as recuperative agents for fatigue, but these supplements are rather meant for nutritional supplements for physical fatigue. What is more, nothing has been known yet about recuperative agents having effects and efficacies for mental fatigue.

Chlorogenic acids have been reported to have, for example, an anti-hypertensive effect, an effect of improving vascular endothelial function, and the like. However, it remains to be reported whether chlorogenic acids is really effective for the recovery of the cerebral fatigue (decreased mental capacity) caused by a long-term exposure to computer or continued brain work (see, for example, Patent Documents 1 and 2).
[Patent Document 1] JP-A-2002-53464
[Patent Document 2] JP-A-2003-261444

### Disclosure of the Invention

The present invention provides a recuperative agent for human cerebral fatigue or a recuperative food for human cerebral fatigue, which contains, as active ingredients, one or more members selected from the group consisting of chlorogenic acids, caffeic acid, ferulic acid, and pharmaceutically acceptable salts thereof.
The present invention also provides use of one or more members selected from the group consisting of chlorogenic acids, caffeic acid, ferulic acid, and pharmaceutically acceptable salts thereof, for the manufacture of a recuperative agent for human cerebral fatigue.
The present invention further provides a method for the recuperation from cerebral fatigue in humans, which includes administering an effective amount of one or more members selected from the group consisting of chlorogenic acids, caffeic acid, ferulic acid, and pharmaceutically acceptable salts thereof.

### Brief Description of the Drawings

Fig. 1 is a graph showing the amount of changes in the flicker test results after taking chlorogenic acids.
Fig. 2 is a graph showing the effects of chlorogenic acids on tedium.
Fig. 3 is a graph showing the effect of chlorogenic acids on active pleasure.
Fig. 4 is a graph showing the amount of changes in the flicker test results after taking chlorogenic acids.
Fig. 5 is a graph showing the effects of chlorogenic acids on tedium.
Fig. 6 is a graph showing the effects of chlorogenic acids on active pleasure.
Fig. 7 is a graph showing the effects of chlorogenic acids on a task of detecting alphanumeric characters.
Fig. 8 is a graph showing the effects of chlorogenic acids on a task of adding numbers.
Fig. 9 is a graph showing the effects of chlorogenic acids on a short-term memory task.

### Mode for Carrying Out the Invention

The present invention provides a recuperative agent for the fatigue caused by mental activities intrinsic to humans (for example, calculating ability, sustained thinking, etc.), that is, a recuperative agent for cerebral fatigue.

The inventors of the present invention conducted a search of an ingredient for the recuperation from a decreased calculating ability due to continued operation, or the like, that is, recuperation from cerebral fatigue, by combining a flicker test which evaluates the degree of cerebral fatigue via optical fatigue, and a computational loading operation which induces cerebral fatigue. As a result, we found that one or more members selected from the group consisting of chlorogenic acids, caffeic acid, ferulic acid, and pharmaceutically acceptable salts thereof have an effect for the recuperation from cerebral fatigue.

According to the present invention, a decreased mental ability exhibited after a long-term use of computer or induced by mental fatigue (cerebral fatigue) due to calculations or other brainactivities, canberecuperatedeffectively. Furthermore, cerebral fatigue can be alleviated by taking the agent or the food prior to any mental activity presumed to cause cerebral fatigue.

The chlorogenic acids, caffeic acid, and ferulic acid used in the present invention may be either extracted from natural products, particularly plants, containing them, or may be industrially produced by chemical synthesis.

The chlorogenic acids, caffeic acid and ferulic acid according to the present invention exist as mixtures of stereoisomers. Thus, in the present invention, pure stereoisomers or mixtures thereof can be used. Examples of the chlorogenic acids according to the invention include 3-caffeylquinic acid, 4-caffeylquinic acid, 5-caffeylquinic acid, 3,4-dicaffeylquinic acid, 3,5-dicaffeylquinic acid, 4,5-dicaffeylquinic acid, 3-ferulylquinic acid, 4-ferulylquinic acid, 5-ferulylquinic acid, 3-ferulyl-4-caffeylquinic acid, and the like (Nakabayashi et al., "Chemistry and Technology of Coffee Roasting", Kogaku Shuppan Co., Ltd., p.166-167).

The chlorogenic acids, caffeic acid and ferulic acid can have their water solubility enhanced and their physiological efficacy increased, when they are salified. For such salts, any pharmaceutically acceptable salts may be used. Examples of basic substances useful for forming such salts include hydroxides of alkali metals such as lithium hydroxide, sodium hydroxide, potassium hydroxide; hydroxides of alkaline earth metals such as magnesium hydroxide, calcium hydroxide; inorganic bases such as ammonium hydroxide; basic amino acids such as arginine, lysine, histidine, ornithine; and organic bases such asmonoethanolamine, diethanolamine, triethanolamine,with the hydroxides of alkali metals and alkaline earth metals being preferred. According to the present invention, these salts may be prepared first and then added to a composition consisting of the remaining ingredients, or alternatively, chlorogenic acids and the like and the ingredients for forming salts may be separately added to the composition and allowed to form salts therewithin.

Preferred examples of natural product extracts containing chlorogenic acids or caffeic acid include extracts of plants such as coffee, cabbage, lettuce, artichoke, tomato, eggplant, potato, carrot, apple, pear, plum, peach, apricot, cherry, sunflower, mulukhiya, sugarcane, nandina leaf, blueberry, wheat.
For example, chlorogenic acids are preferably extracted from plants such as green coffee bean, nandina leaf, unripe apple fruit, and more preferably, the acids are extracted from seeds of Coffee arabica LINNE with a warmed acidic aqueous solution of ascorbic acid or citric acid, or with hot water.

Specifically, there may be mentioned "Flavor Folder" supplied by T. Hasegawa Co., Ltd. as an extract of green coffee bean; "Applephenon" supplied by Nikka Whiskey Distilling Co., Ltd. as an apple extract; "Heliant" supplied by Dainippon Ink and Chemicals Incorporated as a sunflower seed extract; and the like.

Preferred examples of natural product extracts, particularly plant extracts, containing ferulic acid include extracts of coffee, onion, radish, lemon, cnidiumrhizome, maize, pine, coptis root, asafetida, sugarcane, corn, barley, wheat, rice and the like, and among them an extract of rice is preferred. The term "rice" as used herein means raw or dried seeds of Oryza sativa LINNE.

For the method of extracting ferulic acid from plants, a method may be mentioned, for example, in which rice bran oil obtained from rice bran is partitioned between aqueous ethanol and hexane phases in weak alkali at room temperature, and then any ferulic acid ester obtained in the aqueous ethanol fraction is hydrolyzed with sulfuric acid with heating under pressure, and the resultant product then being purified. Ferulic acid may also be obtained by culturing bacteria (Pseudomonas) in a culture solution containing clove oil obtained from buds and leaves of Syzygium aromaticum MERRILL et PERRY by steam distillation, or containing eugenol obtained by purifying clove oil, and then subjecting the resultant culture solution to separation and purification.

Ferulic acid can also be prepared by chemical synthesis, for example, by a condensation reaction of vanillin and malonic acid (Journal of American Chemical Society, 74, 5346, 1952).

The agent for recovery from cerebral fatigue in humans according to the present invention renders recovery from the cerebral fatigue based on human mental activities, and alleviation of the cerebral fatigue. The term "mental activities of humans" refers to anymental activities accompanied by human capacity of logical understanding. Examples of the human mental activities include thinking, calculating, computer operation, and the like. Furthermore, the term "cerebral fatigue based on human mental activities" as described above refers to the state after 1 to 2 hours of PC operation or the like, although there may be differences in individuals.

With regard to the cerebral fatigue based on mental activities, the mental fatigue resulting from the Kraepelin test, ATMT, computational loading operations and the like can be evaluated by performing a flicker test or the like.
The Kraepelin test is an operation involving serial arithmetic additions, which is originally used for the purpose of mental examination. Specifically, the test is an operation of sequentially adding single-digit numbers that are arrayed in adjacent, lateral rows, and can be placed as an operation inducing cerebral fatigue based on heavy operations of mental activities. Here, the Kraepelin test can be utilized as a model system of calculating capacity, computer operation, continuous thinking or the like, for the purpose of quantification.

The ATMT, which is originally used as a mental function test, involves a task for visual search, performed by pressing in order the target numbers displayed on a touch panel. In the test, the search response time for each target can be recorded, and thus, it is possible to rearrange the targets or to create new additional targets, depending on the response. Therefore, it is also possible to measure the working memory or the degree of fatigue while performing a task.

The computational loading operations involve assigning to a test subject a task of detecting alphanumeric characters, a task of adding numbers, and a short-term memory task. The task of detecting alphanumeric characters is a task of clicking the left button of a mouse if a fixed numeric or alphabet character is presented, and clicking the right button if otherwise. The task of adding numbers is a task of adding two-digit numbers presented in two columns altogether and inputting the total through a keyboard. The short-term memory task is a task of clicking the left button of a mouse if numbers corresponding to the four numbers initially presented on the left side are presented on the right side, and clicking the right button of a mouse if numbers not corresponding thereto are presented. These three tasks can be placed as operations inducing cerebral fatigue based on operations of mental activities.

The flicker test is a method of measuring visual responses to a light source flickering at a certain interval, and is used to directly evaluate the activity status (fatigue status) of the cerebral cortex through the organ of vision.

The flicker test is performed to determine the effect of rendering recovery in the degree of cerebral fatigue, and it is further preferable to measure fingertip volume pulse waves (chaos analysis) to measure mental responses. The fingertip volume pulse wave measurement (chaos analysis) allows digitization of the systemic status of the body from the pulse wave data from fingertips, on the basis of the view that the dynamic state of pulses is chaotic. Chaos is characterized by being generated by a plurality of chaoses intertwining to form a localized single chaos. Therefore, it is believed that when an analysis is made using this theory, the systemic state of the body can be recognized in numerical values from the information of a portion of the body.

Furthermore, to investigate the subjective sense of fatigue, it is preferable to use a questionnaire concerning the measure of multifaceted emotional state, examination of subjective symptoms and fatigue.

The effect of the recuperation from fatigue according to the present invention lies in that a decrease in the results of a flicker test induced by mental fatigue (cerebral fatigue) is recovered and alleviated by ingestion of chlorogenic acids and the like.
In addition, the cerebral fatigue that can be recuperated by the agent for recovery from cerebral fatigue in humans is predominantly the fatigue based on the deskwork and the like performed at the scenes of work or study.

The chlorogenic acids and the like, which are the active ingredients of the recuperative agent for human cerebral fatigue according to the present invention, may be directly taken, but preferably can be taken in the form of pharmaceutically acceptable salts, for example, a hydrogen chloride salt, together with auxiliary ingredients such as excipients, carriers that are generally used in the fields of pharmaceuticals and food products, such as lactose, sucrose, syrup, honey, magnesium stearate, oxypropylcellulose, various vitamins, citric acid, malic acid, flavors, inorganic salts, in formulations such as capsules, tablets, powders, granules, healthdrinks, injections, infusions.

In the case of health drinks and food products, it is also possible to add other physiologically active ingredients, minerals, vitamins, hormones, nutrients, flavoring agents and the like according to necessity. Furthermore, the food product is preferably prepared as green tea-based beverages, oolong tea-based beverages, black tea-based beverages, coffee-based beverages, or isotonic drinks.

The recuperative agent for human cerebral fatigue and the recuperative food for cerebral fatigue according to the present invention are useful for the recuperation from a decrease in cerebral function, such as a decrease in the calculating ability mainly based on the use of computer and the like, and the daily dose for an adult is preferably from 30 to 14,000 mg, more preferably from 50 to 10,000 mg, even more preferably from 200 to 7,600 mg, and far more preferably from 250 to 3,000 mg, of chlorogenic acids, caffeic acid, ferulic acid or pharmaceutically acceptable salts thereof. In order to provide the effect of recuperation from cerebral fatigue more effectively, it is believed that it is preferable to continually take the agent or the food everyday. Also, the recuperative food for human cerebral fatigue according to the present invention can be labeled as "for those feeling the cerebral fatigue", "for the recuperation from decreased cerebral function", or the like.

### EXAMPLES

### EXAMPLE 1

Eight normal subjects were entered to a room at constant temperature and humidity and subjected to acclimation for 10 minutes, and then a flicker test was performed with the subj ects in their stable states. Subsequently, computational loading operations (3 minutes for the task of detecting alphanumeric characters, 5 minutes for the task of adding numbers, and 3 minutes for the short-term memory task) were performed, and then another flicker test was performed. After the last flicker test, questions concerning the mental state and the sense of fatigue at that time point were asked using a subjective questionnaire (a brief version for the measure of multifaceted emotional state) . After 5 minutes of rest, the test procedure was repeated in the order of a flicker test, computational loading operations and another flicker test. Thereafter, a beverage having a composition of green coffee bean extract containing chlorogenic acids as the main ingredient (300 mg of chlorogenic acids), or water (placebo) was given to each of the subjects, and another flicker test was performed. At the same time, questions concerning the mental state and the sense of fatigue at that time point were asked before and after drinking the beverage, using a subjective questionnaire. At the respective time points of 30 minutes, 60 minutes, 90 minutes and 120 minutes after drinking the beverage having a composition of green coffee bean extract containing chlorogenic acids as the main ingredient, or water, the subjects were subjected to a flicker test, computational loading operations, another flicker test and a subjective questionnaire in the same manner, and the changes in the scores of the flicker tests after the computational loading operations and the scores of the subjective questionnaires at the respective time points were determined. The subjective questionnaire is a set of questions for simultaneously measuring the subjective state of multiple emotions, and is designed to check the extent to which a subject is feeling about the five emotions of depression/anxiety (worried, anxious, troubled, unconfident, brooding), tedium (uninteresting, tired, bored, weary,languid),active pleasure (vivacious, lively, energetic, vigorous, cheerful), inactive pleasure (laid-back, unhurried, leisurely, tranquil, easy-going), and concentration (cautious, polite, courteous, prudent, careful), in four grades (1. never felt, 2. rarely felt, 3. slightly felt, 4. clearly felt).

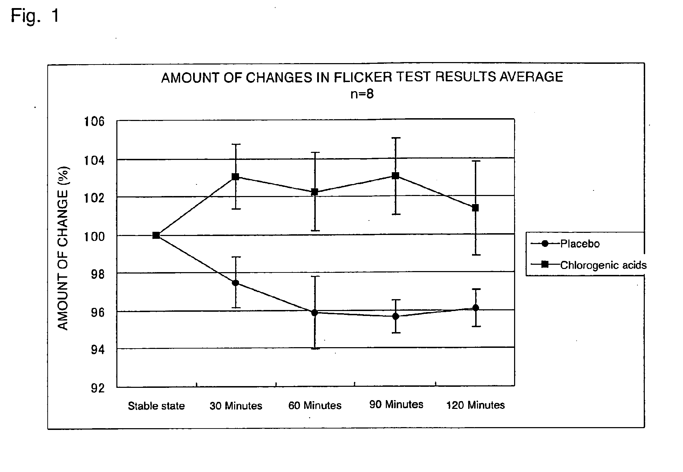

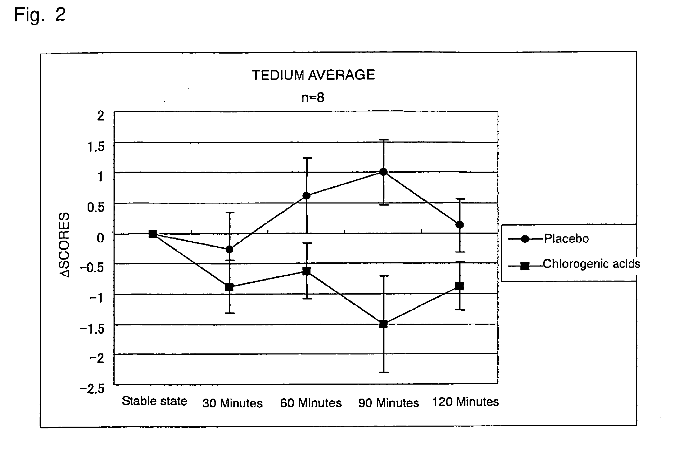

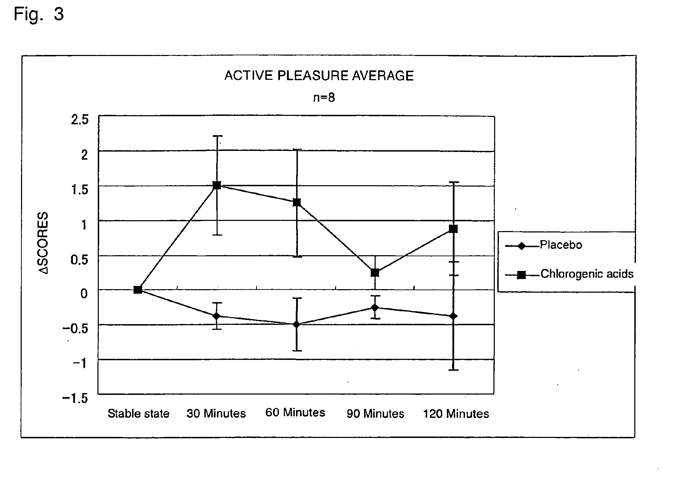

The results of the flicker tests are shown in Fig. 1. When chlorogenic acids were taken, the decrease in the flicker test results was suppressed compared to the case of taking placebo. When the results were examined using two-factor factorial ANOVA, significant differences were recognized in the variation between the samples (placebo and chlorogenic acids) (p < 0.01). Furthermore, when the results were compared using one factor ANOVA and Fisher's PLSD as a multiple comparison test of a hypostasis test, significant differences were recognized in the results obtained at 30 minutes, 60 minutes and 90 minutes after drinking the samples (p = 0.02 , p = 0.04 , p < 0.01). Furthermore, as shown in Figs. 2 and 3 regarding the subjective questionnaires, significant differences were recognized in the variation between the samples (placebo and chlorogenic acids) in the aspects of tedium and active pleasure (p = 0.04, p = 0.05).

### EXAMPLE 2

Eight normal subjects were entered to a room at constant temperature and humidity and subjected to acclimation for 10 minutes, and then a flicker test was performed with the subj ects in their stable states. Subsequently, computational loading operations (3 minutes for the task of detecting alphanumeric characters, 3 minutes for the task of adding numbers, 3 minutes for the short-term memory task, and ATMT) were performed, and then another flicker test was performed. After the last flicker test, questions concerning the mental state and the sense of fatigue at that time point were asked using a subjective questionnaire (a brief version for the measure of multifaceted emotional state). After 5 minutes of rest, the test procedure was repeated in the order of a flicker test, computational loading operations and another flicker test. Thereafter, 1.25 g of a powder of green coffee bean extract containing chlorogenic acids as the main ingredient (600 mg of chlorogenic acids, components: 47.9% of chlorogenic acids and 1.1% of caffeine), or 644 mg of placebo (components: 0.8% of chlorogenic acids and 0% of caffeine) was encapsulated in 5 oblate capsules in total, the capsules were taken with 190 mL of water by each of the subjects, and another flicker test was performed. At the same time, questions concerning the mental state and the sense of fatigue at that time point were asked before and after drinking the beverage, using a subjective questionnaire. At the respective time points of 15 minutes, 30 minutes, 60 minutes, 90 minutes and 120 minutes after taking the green coffee bean extract containing chlorogenic acids as the main ingredient, or water, the subjects were subjected to a flicker test, computational loading operations, another flicker test and a subjective questionnaire in the same manner, and the changes in the scores of the flicker tests after the computational loading operations and the scores of the subjective questionnaires at the respective time points were determined. The subjective questionnaire is a set of questions for simultaneously measuring the subjective state of multiple emotions, and is designed to check the extent to which a subject is feeling about the five emotions of depression/anxiety (worried, anxious, troubled, unconfident, brooding), tedium (uninteresting, tired, bored, weary, languid), active pleasure (vivacious, lively, energetic, vigorous, cheerful), inactive pleasure (laid-back, unhurried, leisurely, tranquil, easy-going), and concentration (cautious, polite, courteous, prudent, careful), in four grades (1. never felt, 2. rarely felt, 3. slightly felt, 4. clearly felt).

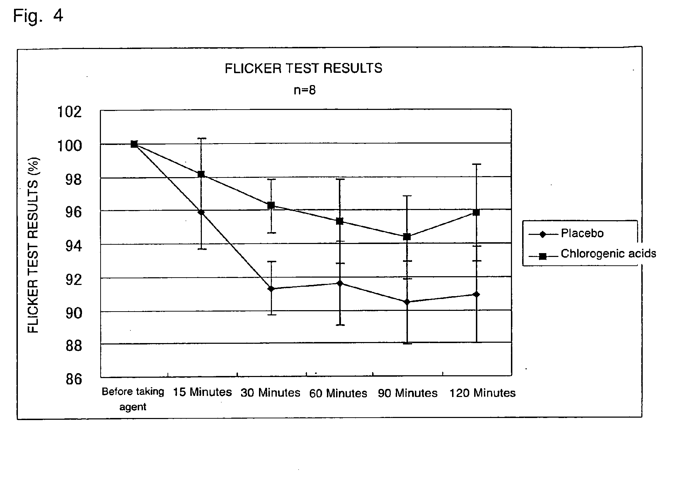

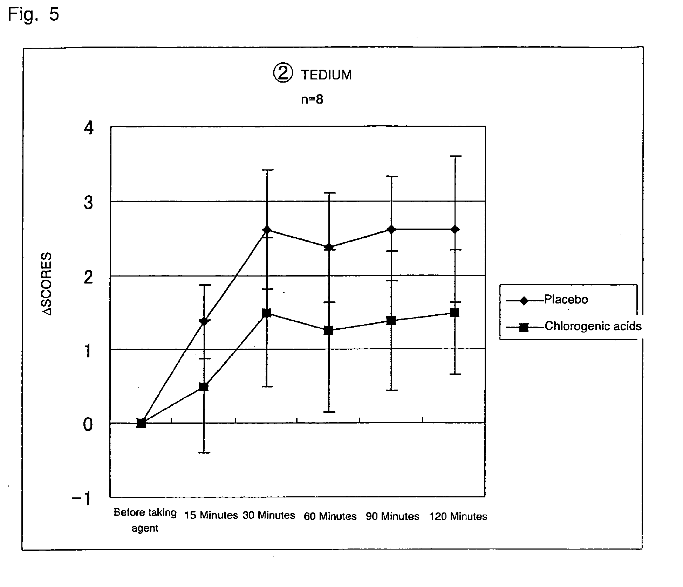

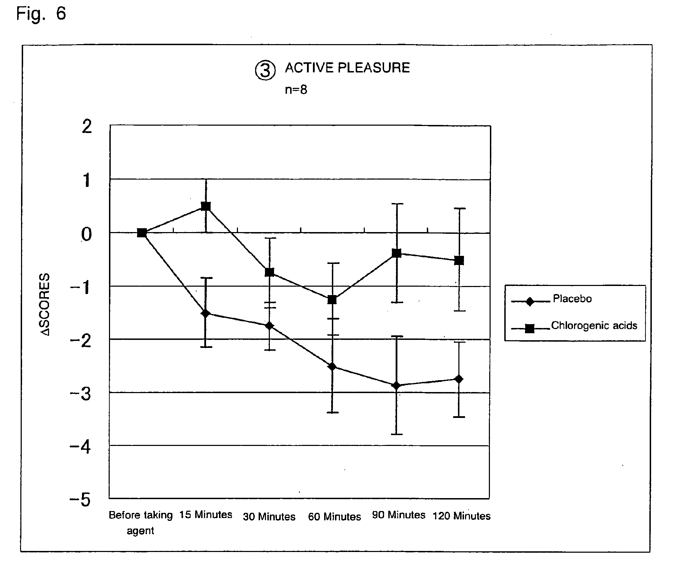

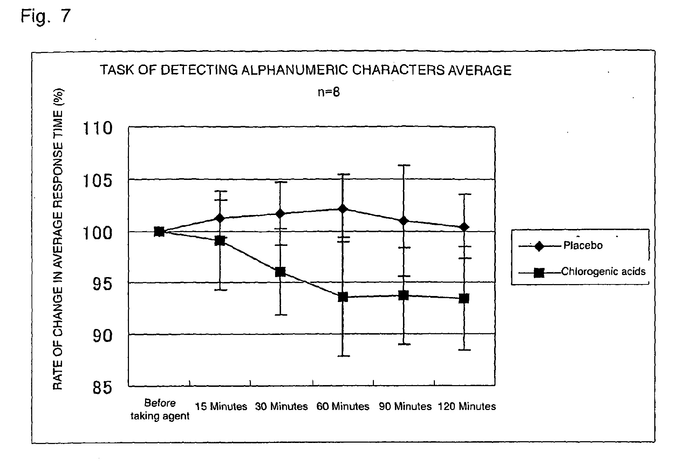

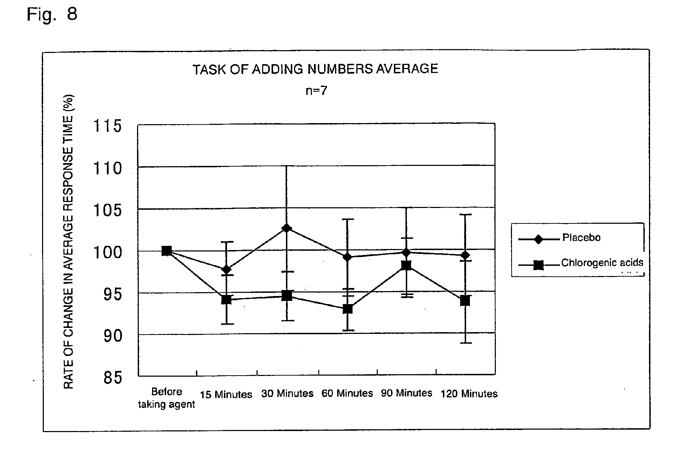

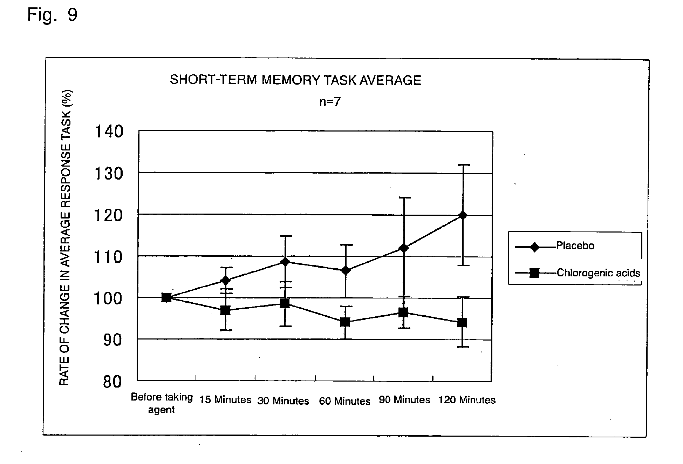

The results of the flicker tests are shown in Fig. 4. When chlorogenic acids were taken, the decrease in the flicker test results was suppressed compared to the case of taking placebo. When the results were examined using two-factor factorial ANOVA, significant differences were recognized in the variation between the samples (placebo and chlorogenic acids) (p<0.01). Also, as shown in Figs. 5 and 6 regarding the subjective questionnaires, significant differences were recognized in the variation between the samples (placebo and chlorogenic acids) (p = 0. 05, p < 0.01) in the aspects of tedium and active pleasure. Furthermore, when the results were compared using Fisher's PLSD as a multiple comparison test of a hypostasis test, significant differences were recognized in the results obtained at 15 minutes after drinking the samples (p = 0.03) in the aspect of active pleasure. Also, with respect to the results obtained at 90 minutes and 120 minutes after drinking the samples, a tendencywas recognized in the difference between the samples (p = 0.08, p = 0.08).
In addition, the results of the task for computational load are presented in Figs. 7, 8 and 9. As shown in the figures, meaningful differences were recognized in the variation between the samples for the task of detecting alphanumeric characters and the short-term memory task (p = 0.03, p < 0.01). Also, a tendency in the difference between the samples was recognized for the task of adding numbers (p = 0.08).

## Claims

1. A recuperative agent for human cerebral fatigue, comprising as active ingredients one or more members selected from the group consisting of chlorogenic acids, caffeic acid, ferulic acid, and pharmaceutically acceptable salts thereof.

2. The recuperative agent for cerebral fatigue according to claim 1, wherein the cerebral fatigue based on mental activities is alleviated thereby.

3. A recuperative food for human cerebral fatigue, comprising as active ingredients one or more members selected from the group consisting of chlorogenic acids, caffeic acid, ferulic acid, and pharmaceutically acceptable salts thereof.

4. Use of one or more members selected from the group consisting of chlorogenic acids, caffeic acid, ferulic acid, and pharmaceutically acceptable salts thereof, for the manufacture of a recuperative agent for human cerebral fatigue.

5. The use according to claim 4, wherein the recuperative agent for cerebral fatigue is an agent for alleviating the cerebral fatigue based on mental activities.

6. A method for recuperating human cerebral fatigue, comprising administering an effective amount of one or more members selected from the group consisting of chlorogenic acids, caffeic acid, ferulic acid, and pharmaceutically acceptable salts thereof to a subject in need thereof.

7. The method according to claim 6, wherein the cerebral fatigue based on mental activities is alleviated thereby.
